# EUROPEAN PATENT APPLICATION

(11) **EP 2 623 143 A1**
(43) Date of publication of application: **07.08.2013**
(21) Application number: 11829662.3
(22) Date of filing: 11.08.2011
(51) Int. Cl.: A61M 5/24, A61M 5/32

(54) **DISPOSABLE CARTRIDGE INJECTOR**

(30) Priority: 29.09.2010 RU 2010139938
(71) Applicant: Abovyan, Aleksandr Ilyich, Tver 170001 (RU); Abovyan, Roman Aleksandrovich, Moscow 109341 (RU); Abovyan, Aleksandr Aleksandrovich, Moscow 109341 (RU)
(72) Inventor: Abovyan, Aleksandr Ilyich, Tver 170001 (RU); Abovyan, Roman Aleksandrovich, Moscow 109341 (RU); Abovyan, Aleksandr Aleksandrovich, Moscow 109341 (RU)
(74) Representative: Staudt, Hans-Peter
(86) International application number: PCT/RU2011/000608
(87) International publication number: WO 2012/044196

(57) **Abstract**

The invention relates to medical equipment, namely, to devices for subcutaneous, intramuscular, intravenous and intraosseous injections of liquid medications placed in carpules used for single-use injections, in particular, for implementation of local anesthesia in dentistry. A disposable carpule injector comprises a cylindrical barrel, which has at one end a handle-finger rest with a hole at the face end for installation, with a possibility of displacement therein, of a rod-rest with a bar-shaped tip, and at the opposite end, a bush for a double-edged needle, a carpule installed in a barrel with a possibility of contact of the carpule plunger and the tip of the rod-rest, and a fastening element with a double-edged injection needle, where bush for the double-edged needle is made with a possibility of threaded fixation of the fastening element with the double-edged injection needle thereon and installation of a protective needle cap, the handle-rest is made with a possibility of being fixed on the barrel by means of projections fixing the barrel and corresponding grooves on the handle-rest, and is provided with a clamp for the carpule, the carpule end opposite to the plunger has a metal cap, the barrel has a pull-out protective cap following the shape of the barrel, installed with a possibility of being moved along the same, extended to the full length of the injection needle and fixed on the barrel by means of fixation elements forming a fixation unit.

## Description

The invention relates to medical equipment, namely, to devices for subcutaneous, intramuscular, intravenous and intraosseous injections of liquid medications placed in carpules used for single-use injections, in particular, for implementation of local anesthesia in dentistry.

In modem dentistry, carpule syringes are used, which have remained virtually unchanged since their invention. Carpule syringes used in dentistry have a heavy metal barrel, they cannot be fixed using the generally accepted "pen grasp" as they are reusable and require sterilization prior to each application. Besides, in the course of inserting the carpule (cartridge) into the syringe, the carpule diaphragm loses sterility and requires additional treatment with antiseptic solution.

Use of disposable injectors made of plastic, which are sterilized in conditions of serial production, partially solves the aforesaid problems of aseptic and antiseptic; however, the medical personnel, by virtue of their direct duties, are constantly subject to high risk of contamination with various contagious diseases, in particular, HIV. Contamination generally takes place at accidental injuries (punctures) with infected needles and most often, in case of attempts to cover a used needle with "a cap", and as a result of cuts during various surgical operations. Laboratory and epidemiological studies suggest that in about 0.3% of cases, contaminations occur at damage related to injuring with an infected needle.

Known is a dental syringe for injection of an anesthetic being in a medical ampoule (RU 2004115638, 2005), where the barrel has a needle part and a rod part, as well as a cavity for an ampoule. With the rod introduced into the barrel, the ampoule is pricked against a poker of a unit containing a needle, and the above unit itself is fixed reliably. The needle has a cap connected to the cap of the poker for complete closure and protection of the unit containing the needle. The syringe is used for performing conduction and infiltration anesthesia into soft tissues, however, its use does not ensure high sterility of the injection system prior to contact with the patient.

Known is a disposable carpule syringe used for anesthesia in dentistry (WO 00/35519, 2000), which comprises a hollow cylindrical barrel, which has finger rests at one of its ends, and an element for attachment of a double-edged injection needle at the other end. Inside the barrel, there is a carpule with a medication in the form of a tubular barrel shut at its face ends with a rubber plunger and a metal cap, this carpule having one of its ends combined with a rod - finger rest. The barrel and the rod-finger rest are made of plastic and are intended for single use, yet the design of the syringe does not protect the medical personnel from injuries inflicted with a used infected needle.

Also known is a carpule syringe (RU 40894, 2004) used in dentistry and considered as a prototype. The syringe comprises a cylindrical barrel with a hole at the front end, a carpule with a plug-plunger and a rod placed inside the cylindrical barrel, with the rod being able to interact with the plug-plunger of the carpule, a double-edged needle and an element for fastening the same at the front end of the carpule. The needle is placed inside the syringe barrel, in which there is a spring installed between the needle fastening element and the front face end of the syringe barrel. The design of the syringe excludes a possibility for the needle to break off the cannula in case of the patient's sudden sharp movements, reduces the patient's stress before the injection and increases sterility of the injection system owing to a smaller number of parts getting in contact with the needle. However, the syringe does not make possible to minimize the probability of injuring the medical personnel accidentally with an infected needle.

The proposed device is aimed at eliminating the aforesaid shortcomings.

The technical result of this invention is increasing sterility of the entire injection system prior to its contact with the patient, minimizing the probability of infecting the medical personnel accidentally, improving the patient's psycho-emotional state through eliminating the stage of assembly and preparation of the syringe-injector for operation right in front of the patient, as well as reducing the risk of injecting the anesthetic solution into a vessel owing to performance of a full aspiration test.

The objective, for which the claimed invention is aimed, is achieved by the fact that in a disposable carpule injector comprising a cylindrical barrel, which has at one end a handle-finger rest with a hole at the face end for installation, with a possibility of displacement therein, of a rod-rest with a bar-shaped tip, and at the opposite end, a bush for a double-edged needle, a carpule installed in a barrel with a possibility of contact of the carpule plunger and the tip of the rod-rest, and a fastening element with a double-edged injection needle, the bush for the double-edged needle is made with a possibility of threaded fixation of the fastening element with the double-edged injection needle thereon and installation of a protective needle cap, the handle-rest is made with a possibility of being fixed on the barrel by means of projections fixing the barrel and corresponding grooves on the handle-rest, and is provided with a clamp for the carpule, the carpule end opposite to the plunger has a metal cap, the barrel has a pull-out protective cap following the shape of the barrel, installed with a possibility of being moved along the same, extended to the full length of the injection needle and fixed on the barrel by means of fixation elements forming a fixation unit.

It is preferable that there would be a numerical scale on the body of the rod-rest for convenience of dosing and control of the medication consumption in the course of injection.

The rubber plunger may have a recess for tight connection with the tip of the rod-rest.

The fixation elements of the fixation unit may be constituted by a groove on the outer side of the barrel on the side of the bush for the double-edged needle, its size corresponding to the projection on the inner side of the pull-out protective cap, made on the side of the handle-rest on the barrel.

Besides, the barrel of the injector may have two movable projections on the side of the bush for the double-edged needle, each of which is attached to the inner surface of the barrel with one end, while the other end is free, and two fixed projections.

It is preferable that the protective cap would be made with a sight window, and the finger rests in the handle-rest would be made in the form of rings.

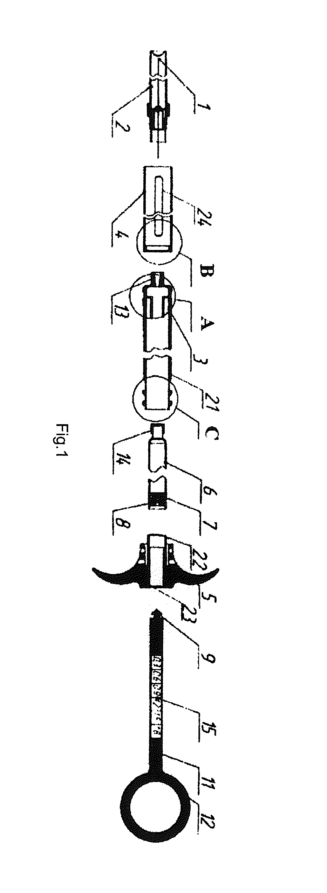

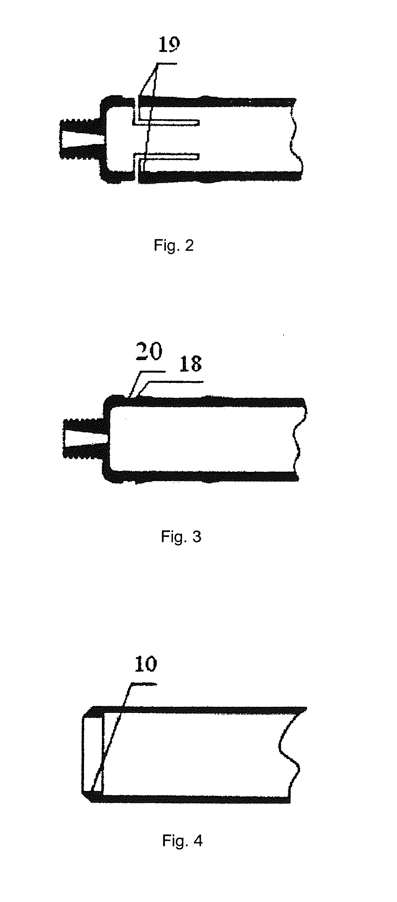

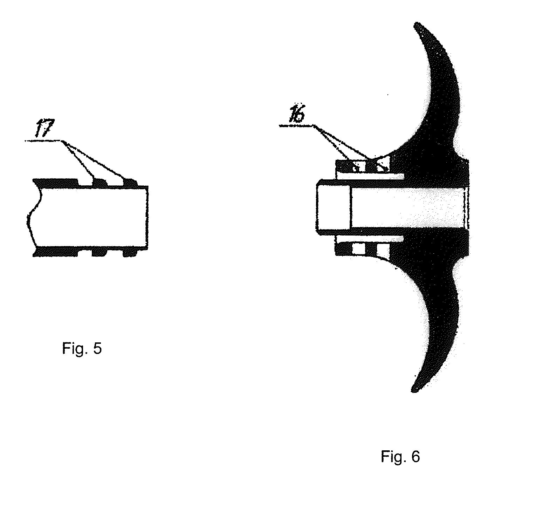

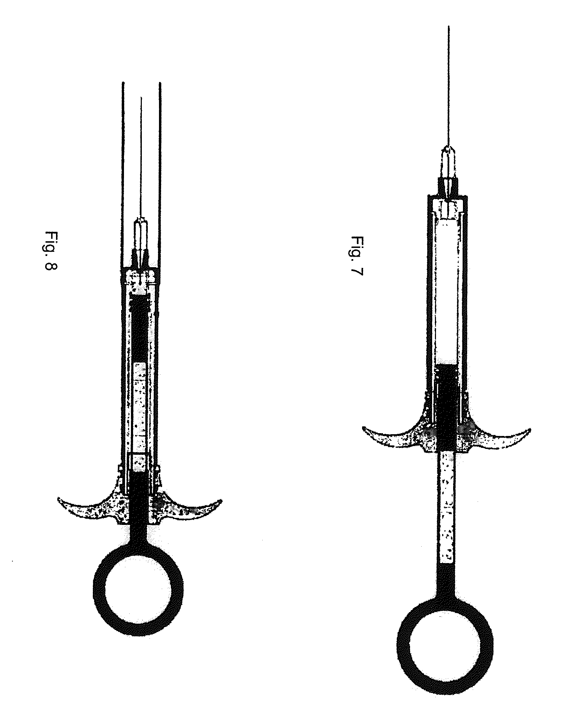

Fig. 1 presents components of the carpule injector; Fig.2, view A of Fig. 1; Fig.3, view A (rotated) of Fig.1; Fig. 4, view B of Fig.1; Fig. 5, view C of Fig.1; Fig. 6, the handle-rest; Fig.7, the carpule injector ready for operation; Fig.8, a view of the carpule injector after injection.

The injector comprises cylindrical barrel 3, on one end there being fixing projections 17 for fixation with handle-rest 5 with holes-grooves for fixation 16 and clamp for carpule 22 with hole 23 for passage of rod-rest 11 with bar-shaped tip 9 on one end and circular finger rest 12 on the other one. The body of rod-rest 11 bears numerical scale for convenience of dosing and control of medication consumption in the course of injection. Handle-rest 5 may have finger rests of various shapes, e.g., circular ones (not shown in pictures).

At the opposite end of barrel 3 there is bush for double-edged needle 13, provided with a thread for installation of a fastening element with double0edged needle 1 and protective needle cap 2.

From the face end of the barrel from the side of fixing projections 17, carpule 6 is inserted shut at the face ends with rubber plunger 7 (from the side of handle-rest of carpule 5) and metal cap 14 (from the side of bush for needle 13). Rubber plunger 7 has recess 8, into which tip 9 of rod-rest 11 goes, whereby their tight connection is achieved, which enables to exercise reciprocating movement of plunger 7 along the carpule barrel; meanwhile, a negative pressure is created inside the carpule, with an aspiration effect arising.

On injector barrel 3, protective cap 4 is fixed with sight window 24, the cap following the barrel shape and being installed with a possibility of being extended to the full length of the injection needle. Protective cap 4 is installed with a possibility of functioning in 2 modes: the initial one - fixed with projection 21, for excluding the possibility of spontaneous displacement of the protective cap, and the "final" one - for termination of work with the injector. With protective cap 4 extended to the maximum, the design of the device prevents it from returning to the initial position owing to a mechanism in the cap fixation unit comprising of groove 20 on the outer side of the injector barrel on the side of the bush for the double-edged needle, its size corresponding to projection 10 on the inner side of the barrel of the pull-out protective cap, made on the side of the injector barrel fixing elements. Here, cap 4 is extended to the full length of the injection needle, is fixed and prevents the device from being used again. Extending, the protective cap protects the injection needle from contact with surrounding objects and eliminates the possibility of injuring the medical personnel accidentally with an infected needle (the mode of "final fixation" of the protective cap).

Besides, the injector barrel has, on the side of the bush for the double-edged needle, two movable projections 19, each of which has one of its ends attached to the inner surface of the barrel, its other end being free, and two fixed projections on barrel 18, which ensures a rest for the pull-out protective cap at its final position - being closed in the fixing unit.

With the carpule passing deeper into the injector barrel, its metal cap stops at the face end of the injector barrel from the side of the double-edged needle; meanwhile, there occurs expansion of both of the projections outwards owing to the carpule diameter; at the same time, from the side of the barrel fixing projections, the carpule is pressed against the face end of the barrel with the handle-rest, barrel fixing projections 17 of the injector come into the hole of handle-rest 16, and finds itself fixed in the injector owing to the rest on the handle-rest.

With the protective cap extended and fixed in the final position, its edge is pressed against a ledge created by both the projections at expansion. Therefore, reverse movement of the protective cap is arrested.

Besides, the protective cap is a movable element and has three positions; in the first, non-working position, this is located on the injector barrel following the latter's shape and is fixed with projection 21 preventing it from spontaneous displacement at extension of the injection needle aside; in the second preliminary position its fixation in the fixing unit is not implemented; with the protective cap extended forward, to the third position to the full length of the injection needle, when it covers the needle completely, the protective cap is completely fixed, which prevents the injector from being used again.

Working with the claimed injector is exercised as follows:

The carpule constitutes a glass vessel containing a medication, closed from one end with a rubber cover and a metal cap with a hole directed towards the location of the threaded element for attachment of a double-edged needle. The double-edged needle is attached to the injector barrel with a threaded joint. On the other side of the carpule, there is a rubber plunger with a recess to take the rod-rest. Coming into the rubber plunger, the rod-rest passes through a pressing bush. The tight connection of the plunger and the rod tip is implemented with a possibility for the rubber plunger to reciprocate along the cylindrical barrel of the carpule.

With pressure exerted onto the rest of the bar-shaped rod, its tip comes into the plunger; at the same time, the latter starts moving into the face end of the barrel, thus pushing the medication out. With the rod rest moving in the reverse direction, the rubber plunger moves to the opposite side owing to tight connection between the plunger and the rod tip creating a negative pressure inside the cylindrical carpule. This enables to perform an aspiration test.

On completion of the injection procedure, protective cap 4 should be extended forward; passing through a projection on the injector barrel made with a possibility of inflection,
with movable projection 19 and fixed projection 18 on the barrel, it comes into groove 20 and is fixed, while the injection needle finds itself inside the protective cap.

The disposable injector is manufactured by extrusion molding of its individual components and subsequent assembling, packing and sterilization of the finished product (injector).

## Claims

1. A disposable carpule injector comprising a cylindrical barrel, which has at one end a handle-finger rest with a hole at the face end for installation, with a possibility of displacement therein, of a rod-rest with a bar-shaped tip, and at the opposite end, a bush for a double-edged needle, a carpule installed in a barrel with a possibility of contact of the carpule plunger and the tip of the rod-rest, and a fastening element with a double-edged injection needle, **characterized in that** the bush for the double-edged needle is made with a possibility of threaded fixation of the fastening element with the double-edged injection needle thereon and installation of a protective needle cap, the handle-rest is made with a possibility of being fixed on the barrel by means of projections fixing the barrel and corresponding grooves on the handle-rest, and is provided with a clamp for the carpule, the carpule end opposite to the plunger has a metal cap, the barrel has a pull-out protective cap following the shape of the barrel, installed with a possibility of being moved along the same, extended to the full length of the injection needle and fixed on the barrel by means of fixation elements forming a fixation unit.

2. The injector according to claim 1 **characterized in that** the body of the rod-rest bears a numerical scale for convenience of dosing and control of medication consumption in the course of injection.

3. The injector according to claim 1 **characterized in that** the rubber plunger of the carpule has a recess for tight connection with the tip of the rod-rest.

4. The injector according to claim 1 **characterized in that** fixation elements of the fixation unit may be constituted by a groove on the outer side of the barrel on the side of the bush for the double-edged needle, its size corresponding to the projection on the inner side of the pull-out protective cap, made on the side of the handle-rest on the barrel.

5. The injector according to claim 1 **characterized in that** the barrel of the injector has two movable projections on the side of the bush for the double-edged needle, each of which is attached to the inner surface of the barrel with one end, while the other end is free, and two fixed projections.

6. The injector according to claim 1 **characterized in that** the protective cap is made with a sight window.

7. The injector according to claim 1 **characterized in that** the handle-rest has finger rests in the form of rings.
